# EUROPÄISCHE PATENTSCHRIFT

(11) **EP 2 517 666 B1**
(45) Veröffentlichungstag und Bekanntmachung des Hinweises auf die Patenterteilung: **05.04.2017**
(21) Anmeldenummer: 12156112.0
(22) Anmeldetag: 20.02.2012
(51) Int. Cl.: A61B 90/50, A61B 17/00, A61B 17/28

(54) **Vorrichtung zum zeitweiligen Halten eines Gegenstandes**
Device for temporarily holding an object
Dispositif de maintien intermittent d'un objet

(30) Priorität: 22.02.2011 DE 102011012419
(43) Veröffentlichungstag der Anmeldung: 31.10.2012
(73) Patentinhaber: Karl Storz GmbH & Co. KG, 78532 Tuttlingen (DE)
(72) Erfinder: Mischnick, David, 12103 Berlin (DE); Preradovic, Oliver, 13629 Berlin (DE); Bühs, Florian, 10777 Berlin (DE); Schnee, Detlev, 13469 Berlin (DE)
(74) Vertreter: Witte, Weller & Partner Patentanwälte mbB

(56) Entgegenhaltungen:
- FR-A1- 2 937 308
- SU-A1- 1 309 930
- US-A- 1 502 890
- US-A- 5 123 410
- US-A1- 2008 083 306
- US-A1- 2009 293 435
- US-B1- 6 382 576

## Beschreibung

Die Erfindung betrifft eine Vorrichtung zum zeitweiligen Halten eines Gegenstandes, der ein relativ durchmessergrößeres Hand- oder Kopfstück aufweist, von dem sich ein langerstreckter durchmessergeringerer Schaft forterstreckt, insbesondere zum Halten eines medizinischen Instrumentes, mit zumindest zwei Klemmelementen zwischen die der Gegenstand bringbar ist, wobei zumindest ein Klemmelement auf das andere Klemmelement zubewegbar ist, so dass der Gegenstand zwischen den Klemmelementen führbar und/oder fixierbar ist, wobei das zumindest eine bewegbare Klemmelement als expandierbarer elastischer Körper ausgebildet ist, der mit einem Fluid beaufschlagbar ist, und mit einer Steuerung zum Steuern der Beaufschlagung des zumindest einen Körpers mit dem Fluid zum Expandieren bzw. Kollabieren des Körpers, wobei sich der expandierbare Körper aufgrund seiner Elastizität und Bereiche der Außenseite des zu haltenden Gegenstandes anschmiegt und sich somit dessen Außenkontor anpasst.

Eine derartige Vorrichtung ist aus der US 6 382 576 B1 bekannt, die die Merkmale des Oberbegriffs von Anspruch 1 offenbart.

Bei dieser Vorrichtung weist ein Gehäuse seitlich eine in Schwerkraftrichtung von oben nach unten durchgehende Einkerbung oder Schlitz auf, die bzw. der zu einer Außenseite offen ist.

In diese Einkerbung kann ein schaftartiger Körper eines bestimmten Durchmesers eingebracht werden. In einer Wand der Einkerbung ist ein aufblähbares Klemmelement angeordnet, das nach Einbringen des schaftartigen Körpers in die Einkerbung aufgebläht werden kann. Dabei expandiert dieses Element und legt sich aufgrund seiner Elastizität um Bereiche der Außenseite des zu haltenden Gegenstandes an.

Der expandierbare Körper ist mit einem Fluid beaufschlagbar, das über eine Steuerung zu bzw. abführbar ist. Ist das Fluid abgeführt, kollabiert der elastische aufblähbare Körper und der zu haltende Gegenstand kann wieder von der Vorrichtung entnommen werden.

Aus der US 2008/0083308 A1 ist eine Vorrichtung zum Halten eines langerstreckten schaftartigen Körpers, im Ausführungsbeispiel eine Zahnbürste bekannt, die zwischen mehrere aufblähbare Körper gebracht werden kann. Dabei können auch mehrere in axialem Abstand nebeneinander angeordnete mehrere aufblähbare Körper vorhanden sein.

Viele Gegenstände, insbesondere medizinische Instrumente, weisen einen langerstreckten Körper auf, der meist einen relativ langen dünnen Schaft und proximalseitig ein demgegenüber durchmessergroßen Hand- oder Kopfstück aufweist.

Es besteht ein Bedarf solche Gegenstände, insbesondere medizinische Instrumente, mittels einer Vorrichtung in einer bestimmten Stellung bzw. Ausrichtung zeitweilig zu halten. Bei einem endoskopischen Instrument kann durch das Instrument hindurch eine visuelle Beobachtung durchgeführt werden, sei es mit dem bloßen Auge oder mittels einer Kamera, die ein Bild auf einen Monitor überträgt.

In der weit verbreiteten minimal-invasiven Chirurgie wird ein solches Instrument in eine Körperhöhle eingeführt und es wird eine ganz bestimmte Stelle im Körper, bspw. ein zu entfernendes Geschwür, anvisiert.

Dabei führt der Operateur das Endoskop zunächst in den Körper ein, richtet aus, bis er bspw. genau diese beobachtende Stelle erfasst hat. Anschließend soll das endoskopische Instrument genau in dieser Stellung verbleiben, so dass bspw. über eine Kamera auf einem Monitor permanent ein Bild ersichtlich ist, das den Ort des operativen Eingriffes darstellt.

Für diesen Eingriff muss der Operateur dann andere Instrumente bedienen, so dass er das endoskopische Beobachtungsinstrument nicht in einer bestimmten Orientierung halten kann.

Dazu sind die eingangs erwähnten Vorrichtungen zum zeitweiligen Halten eines solchen Gegenstandes vorgesehen. Insbesondere bei dem zuvor erwähnten Einsatz eines endoskopischen Instrumentes, so dass sich die Lage des durch die Vorrichtung zeitweilig gehaltenen Instrumentes möglich nicht verändern, denn schon durch geringe Lageveränderungen könnte die anvisierte Stelle nicht mehr ersichtlich sein. Bei sehr langen und schlanken Elementen ist eine Befestigung an einem relativ dünnen Schaft mit der Gefahr verbunden, dass Kippbewegungen resultieren, insbesondere wenn an einem Ende des Gegenstands relativ bulkige und schwere Baugruppen vorhanden sind. Bei einem Endoskop befinden sich oftmals am proximalen Griff- oder Kopfteil mehrere seitlich abstehende Anschlüsse, bspw. zum Zu- und Abführen von gasförmigen oder flüssigen Medien, die ein beachtliches Gewicht aufweisen und daher bei schrägen Lagen des zu haltenden Gegenstandes, ein erhebliches Kippmoment ausüben.

Daher ist es wünschenswert, einen solchen Gegenstand unmittelbar an dem größeren und auch meist schweren Bauteil, also an dem proximalen Kopf- oder Handstück zu halten.

Bei der eingangs genannten Vorrichtung US 6 382 567 B1 besteht aufgrund der vertikalen Ausrichtung der seitlichen Einkerbung in der das schaftartige Instrument gehalten werden soll, die Gefahr, dass beim Kollabieren des Klemmelements der zu haltende Gegenstand nach unten durchrutscht, insbesondere wenn die Breite des Spaltes so ausgebildet ist, dass dieser den zu haltenden Gegenstand am durchmessergrößeren Kopf einklemmen kann.
Es ist daher Aufgabe der vorliegenden Erfindung hier Abhilfe zu schaffen und eine Vorrichtung zum zeitweiligen Halten eines Gegenstandes der eingangs genannten Art dahingehend weiterzuentwickeln, dass der Gegenstand auch bei kollabiertem Klemmelement haltbar ist.

Die Erfindung ist im unabhängigen Anspruch 1 definiert. Bevorzugte Ausführungsformen sind in den abhängigen Ansprüchen definiert. Erfindungsgemäß wird die Aufgabe dadurch gelöst, dass, bei nicht expandiertem Klemmelement, ein Abstand zwischen den Klemmelementen derart ist, dass auch das durchmessergrößere Hand- oder Kopfstück des zu haltenden Gegenstandes zwischen die Klemmelemente bringbar ist, und dass, in Schwerkraftrichtung gesehen, unter den Klemmelementen eine Abrutschsicherung vorhanden ist, die, bei nicht expandiertem Klemmelement, ein Abrutschen des Gegenstandes von der Vorrichtung dadurch sperrt, dass das Hand- oder Kopfstück zurückhaltbar ist.

Diese Maßnahme hat den Vorteil, dass bei einer Störung oder einem Ausfall der Beaufschlagung mit dem Fluid, der zu haltende Gegenstand nicht von der Vorrichtung abrutscht. Bei einem empfindlichen Gegenstand kann somit verhindert werden, dass nach einem Abrutschen dieser auf einen Boden fällt und beschädigt wird.

Durch Vorsehen eines Klemmelements in Form eines expandierbaren elastischen Körpers kann dieser durch Expansion an den zu klemmenden bzw. haltenden Gegenständen heranbewegt werden und diesen dadurch gegen das andere Klemmelement drücken. Aufgrund der Elastizität schmiegt sich der Körper um Bereiche der Außenseite des zu haltenden Gegenstands an, passt sich somit dessen Außenkontur an. Dies eröffnet die Möglichkeit eine große Bandbreite an Gegenständen unterschiedlicher Größe und unterschiedlicher Geometrie in der Vorrichtung zu halten. Das Fluid, das den elastischen Körper expandiert, sorgt für den ausreichenden Anpressdruck.

Nimmt man als Beispiel ein Griffstück heran, das unterschiedlich geformte Griffmulden zum Einlegen von Fingern hat, so kann sich der elastische Körper dieser Kontur anpassen, also sich in die Fingermulden hineinerstrecken, so dass ein relativ großflächiger formschlüssiger Eingriff zwischen dem elastischen Körper und der Außenseite des zu haltenden Gegenstandes erfolgt.

Das Fluid, das gasförmig, z.B. Druckluft oder flüssig, bspw. Wasser oder ein Öl sein kann, sorgt für die ausreichende Kraft, um den Gegenstand gegen das andere Klemmelement zu drücken und zugleich die Anschmiegung des elastischen Körpers an die Außenkontur des zu haltenden Gegenstandes zu bewerkstelligen.

Die Handhabung ist deswegen besonders einfach, da der Gegenstand lediglich zwischen die beiden Klemmelemente gebracht werden muss und dann das zumindest eine expandierbare Klemmelement durch das Fluid expandiert wird. Dies erfolgt über die Steuerung. Es brauchen also nicht mehr Handhabungsmaßnahmen wie das mehrfache Verdrehen einer Stellschraube oder dergleichen durchgeführt werden. Dasselbe gilt selbstverständlich für das Lösen des Gegenstandes aus der Vorrichtung, hier muss lediglich die Druckbeaufschlagung des Fluids aufgehoben werden, so dass dann der elastische Körper kollabiert, wonach der Gegenstand einfach von der Vorrichtung abgenommen werden kann.

Durch die relativ großflächige Anlage des elastischen Körpers an der Außenseite des zu haltenden Gegenstandes, reicht schon eine relativ geringe Anpresskraft aus, um den Gegenstand in einer ganz bestimmten räumlichen Ausrichtung zu halten.

Dieser relativ geringe Anpressdruck eröffnet auch die Möglichkeit, dass der Gegenstand in der Vorrichtung, falls dies erwünscht ist, bewegt werden kann. Wird bspw. ein Gegenstand an einem langen Schaft mit kreisförmigem Querschnitt gehalten, ist es möglich, den Gegenstand in der Vorrichtung zu drehen oder ggf. axial längs des Schaftes hin und her zu bewegen, wobei er seine prinzipielle geometrische Ausrichtung beibehält. Dies ist bspw. vorteilhaft, wenn man Endoskope einsetzt, die eine von der Mittellängsachse des Schaftes abweichende Blickrichtung aufweisen. Durch Drehen des Schaftes in der Vorrichtung, kann man nun Bereiche um das Operationsfeld herum zusätzlich einsehen. Durch axiales Verschieben kann eine Fokussierung bzw. ein kleineres oder größeres Sichtfeld der Operationsstelle beobachtet werden, ohne dabei die prinzipielle räumliche Ausrichtung des Gegenstandes zu verändern.

Dies ist als ein besonderer Vorteil zu betrachten, dass also nicht nur ein Gegenstand sehr einfach und sicher gehalten werden können, sondern, dass noch gewisse Bewegungen, die sinnvoll und erwünscht werden, möglich sind.

In einer weiteren Ausgestaltung der Erfindung ist der Körper als Hohlkörper ausgebildet, dessen Innenraum mit dem Fluid beaufschlagbar ist.

Diese Maßnahme hat den Vorteil, dass der Expandiervorgang sehr einfach zielgerichtet und sicher durchgeführt werden kann.

Im kollabierten Zustand ist das Fluid aus dem Innenraum des Hohlkörpers abgeführt, so dass der Gegenstand in die Vorrichtung eingeschoben oder aus dieser entnommen werden kann. Durch Einführen des Fluids in diesen Hohlkörper wird dieser expandiert und dabei zielgerecht auf das andere Klemmelement hinzubewegt, sei es durch ein Verschieben, sei es durch Aufblähen des gesamten Hohlkörpers.

Das Fluid beaufschlagt nur den Innenraum des Hohlkörpers, so dass allfällige Kontaminationen nicht in diesen Verstellmechanismus treten können. Bei einem feingängigen Gewinde, bei den Vorrichtungen der eingangs genannten Art, stellen die Gewindegänge Bakteriennischen dar, die nur sehr schwierig und aufwändig zu reinigen sind.

Ferner ist es in dem medizinischen Bereich weit verbreitet, solche Gerätschaften während eines operativen Eingriffes mit einem Überzug (Drape) zu versehen, also bspw. mit einer Kunststofffolie zu bedecken oder zu umwickeln. Bei der Vorrichtung der eingangs genannten Art mit den bewegten Stellschrauben besteht die Gefahr, dass ein solcher Überzug durch die Stellschraube verletzt wird und Kontaminationen doch den Schutzüberzug hindurchtreten können.

Da solche flexible Folien ebenfalls flexible Körper darstellen, können diese problemlos in Zusammenhang mit der erfindungsgemäßen Vorrichtung eingesetzt werden. In anderen Worten ausgedrückt, können die Klemmelemente mit einem solchen Drape überzogen werden, da diese Folien der Expandierbewegung des Körpers folgen können, ohne dass die Gefahr besteht, dass Einrisse oder dergleichen auftreten.

Es können sowohl die Vorrichtung als auch der zu haltende Gegenstand mit einem solchen Drape versehen sein, da der expandierbare Körper sowohl eine Folie, die ihn selbst umgibt, als auch eine Folie, die den zu haltenden Gegenstand umgibt, entsprechend fest an den jeweiligen Körper bzw. Klemmelement anlegt. Dies ist als ein beachtlicher Vorteil der Vorrichtung im medizinischen Bereich anzusehen.

In einer weiteren Ausgestaltung der Erfindung ist der expandierbare elastische Körper als Kissen ausgebildet, das an einem Sitz in einem Rahmen gehalten und/oder geführt ist.

Diese Maßnahme hat den Vorteil, dass das Kissen fest an dem Sitz befestigt werden kann und dann durch das Fluid entsprechend bewegt, oder aufgebläht werden kann. Durch den Rahmen bewegt sich das Kissen zielgerichtet auf das gegenüberliegende Klemmelement beim Beaufschlagen mit Fluid zu.

Dieses erleichtert weiter die einfache Handhabung der Vorrichtung, insbesondere auch das Einsetzen oder Einbringen des Gegenstandes bei nicht expandiertem Kissen.

In einer weiteren Ausgestaltung der Erfindung sind beide Klemmelemente als expandierbare elastische Körper ausgebildet.

Diese Maßnahme hat nun den erheblichen Vorteil, dass der Gegenstand deswegen besonders fest und sicher gehalten ist, da sich von beiden Seiten her elastisch expandierbare Körper an diesen anlegen. Bei dieser Ausgestaltung braucht dann keine Achtung aufgebracht werden, dass der Gegenstand an ein unbewegliches Klemmelement schon in einer bestimmten Orientierung angelegt wird, um auszuschließen, dass dann beim Heranbewegen des beweglichen Klemmelements, sich die Lage bzw. die Anlageorientierung an dem feststehenden Element durch Verdrehen oder Verkippen noch verändert.

Es braucht lediglich der Gegenstand zwischen die beiden Klemmelemente gebracht werden und von beiden Seiten schmiegen sich dann die expandierenden elastischen Körper an.

Dadurch können bspw. Gegenstände, die einen relativ dünnen Schaft aufweisen, sehr lagestabil gehalten werden, wobei die eingangs genannte Möglichkeit des Drehens des Schaftes oder eines axialen Verschiebens nach wie vor frei bleibt.

In einer weiteren Ausgestaltung der Erfindung sind die beiden expandierbaren elastischen Körper an gegenüberliegenden, im Abstand zueinander angeordneten Sitzen, befestigt.

Diese Maßnahme hat den erheblichen Vorteil, dass die Sitze an sich sehr stabil und unbeweglich ausgebildet werden können. Dadurch entfallen auch gelenkartige Verbindungen mit Schwenkzapfen, die insbesondere im medizinischen Bereich problematische Bakteriennischen darstellen. Die Sitze stellen auch ein Widerlager gegen den Anpressdruck dar.

Der Abstand zwischen den beiden expandierbaren Körpern, bzw. den entsprechenden Sitzen, kann dann so gewählt werden, dass eine sehr große Bandbreite an unterschiedlichen Größen an zu haltenden Gegenständen zwischen die Sitze gebracht werden können. Durch entsprechendes Expandieren der elastischen Körper, wird dann der Gegenstand zwischen diesen gehalten. Bei einem relativ durchmessergroßen und bulkigen Gegenstand, müssen dann die Körper nur über einen relativ geringen Weg expandiert werden, bei schlankeren oder dünneren Geräten entsprechend weiter. Dies zeigt eindrucksvoll die variable Einsatzbreite einer einzigen Vorrichtung für unterschiedlich große und unterschiedlich geometrisch ausgestaltete Gegenstände.

In einer weiteren Ausgestaltung der Erfindung, ist der expandierbare elastische Körper eines Klemmelements aus mehreren einzelnen expandierbaren elastischen Körpern aufgebaut.

Diese Maßnahme hat den Vorteil, dass diese einzelnen expandierbaren elastischen Körper eine noch bessere Anschmiegung an besonders konturierte Gegenstände erlaubt. Bei einem einzigen Körper könnte, bei einem extrem konturierten oder sehr schlanken Körper seitliche Ausbauchungen entstehen, die nicht zur Halterung dienen. Durch die Unterteilung in mehrere Einzelkörper ist ein solches Ausweichen begrenzter.

In einer weiteren Ausgestaltung der Erfindung ist jeder der mehreren einzelnen expandierbaren elastischen Körper, unabhängig von einem anderen, über die Steuerung mit dem Fluid beaufschlagbar.

Diese Maßnahme hat den Vorteil, dass eine besonders fein abgestimmte und individuelle Anpassung an unterschiedliche Geometrien von zu haltenden Gegenständen möglich ist, da jeder expandierbare Körper einzeln ansteuerbar ist.

In einer weiteren Ausgestaltung der Erfindung, weist die Vorrichtung einen U-förmigen Grundkörper auf, und die beiden Klemmelemente sind an gegenüberliegenden Innenseiten der Schenkel des U angebracht.

Dies erlaubt eine kompakte und robuste Bauweise der Vorrichtung. Die offene Seite des U bietet dem Benutzer eindeutig die Richtung an, in der er den Gegenstand einbringen bzw. einsetzen soll.

In einer weiteren Ausgestaltung der Erfindung weist die Abrutschsicherung am U-förmigen Grundkörper eine, in Schwerkraftrichtung gesehen, untere Platte auf, die eine Öffnung aufweist, durch die schaftartige Bauteile eines Gegenstandes hindurchreichen, durchmessergrößere Bauteile jedoch zurückhaltbar sind.

Diese Maßnahme hat den Vorteil, dass eine besonders einfache mechanische Halterung gegenüber Abfallen in Schwerkraftrichtung geschaffen ist. Diese eröffnet die Möglichkeit, insbesondere Gegenstände, die an einem Ende einen relativ bulkigen Körper bspw. einen Griff aufweisen, in diesem Bereich zu halten, den verbleibenden Körper aber über die Öffnung aus der Vorrichtung abzuführen.

In einer weiteren Ausgestaltung der Erfindung, ist die Öffnung als ein zur offenen Seite des U's des Grundkörpers öffnender Schlitz ausgebildet.

Diese Maßnahme hat den Vorteil, dass der Gegenstand von der offenen Seite des U her in den Schlitz der Vorrichtung eingeschoben werden kann, wobei das so durchgeführt wird, dass ein bulkiger Körper, bspw. ein Handgriff, zwischen die Klemmelemente gebracht wird, ein langerstreckter Schaft oder Stiel durch den Schlitz bzw. die Schlitzöffnung aus der Vorrichtung vorragen kann, insbesondere in Schwerkraftrichtung gesehen, nach unten. Dies erleichtert ebenfalls die Handhabung und sorgt für eine sichere Halterung auch bei nicht expandierten elastischen Körpern.

In einer weiteren Ausgestaltung sind die Schenkel des Grundkörpers durch eine Wand geschlossen.

Diese Wand stellt eine zusätzliche Abstütz- oder Anlagefläche dar, so dass ein in der Vorrichtung gehaltener Gegenstand an drei Stellen anliegt bzw. von Bauteilen der Vorrichtung gehalten ist. Das sind einmal die beiden gegenüberliegenden Eingriffstellen der expandierten Elemente sowie die dazu sich quer erstreckende Wand, die ein Ende der Schenkel des U verbindet.

In einer weiteren Ausgestaltung der Erfindung, sind alle mit dem Fluid beaufschlagbare expandierbare elastische Körper über Leitungen mit einer einzigen Quelle des Fluids verbunden.

Diese Maßnahme hat den Vorteil, dass nur eine einzige Quelle oder ein einziges Vorratsgefäß für das Fluid bereitgestellt werden muss. Dies eröffnet auch die Möglichkeit, alle mit dem Fluid beaufschlagbare expandierbare Körper über diese Leitungen miteinander kommunizieren zu lassen.

Das eröffnet bspw. die Möglichkeit, nach einem ausgänglichen Halten des Körpers, durch teilweises Kollabieren eines Körpers auf einer Seite durch Abführen von Fluid und gleichzeitigem Zuführen und weiteren Expandieren eines gegenüberliegenden Körpers einen in der Vorrichtung gehaltenen Körper in seiner Lage zu verändern. Dadurch können bspw. Feinausrichtungen und Feinjustierungen durchgeführt werden, indem bspw. ein zwischen den Körpern gehaltener Gegenstand seitlich versetzt oder gekippt wird, oder beide Bewegungen überlagert werden. Damit funktioniert die Vorrichtung nicht nur als reine Halterung, sondern auch zugleich, wenn auch nur in einem bestimmten Maße, als Ausrichtvorrichtung für den Gegenstand.

In einer weiteren Ausgestaltung der Erfindung, ist durch die Steuerung über Ventile jeder der elastischen Körper, unabhängig von einem anderen, mit dem Fluid beaufschlagbar.

Dadurch kann ganz gezielt jeder einzelne elastische expandierbare Körper mit entsprechenden Fluidmengen beaufschlagt oder diese von dem abgeführt werden, um die zuvor erwähnten Feinausrichtungen zu bewerkstelligen.

In einer weiteren Ausgestaltung der Erfindung, weist die Steuerung ein Steuerelement auf, über das alle expandierten Körper zugleich kollabierbar sind.

Diese Maßnahme hat den erheblichen handhabungstechnischen Vorteil, dass über dieses einzige Steuerelement zugleich alle Körper entspannt bzw. kollabieren können, so dass ein Gegenstand sehr einfach und rasch aus der Vorrichtung entnommen werden kann.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Die Erfindung wird in Zusammenhang mit den beiliegenden Zeichnungen anhand einiger ausgewählter Ausführungsbeispiele näher beschrieben und erläutert. Es zeigen:
- Fig. 1: eine perspektivische Darstellung eines ersten Ausführungsbeispiels einer erfindungsgemäßen Vorrichtung mit noch nicht expandiertem elastischen Körper,
- Fig. 2: eine entsprechende Ansicht mit einem expandierten elastischen Körper, der als bewegliches Klemmelement funktioniert,
- Fig. 3: eine etwas vereinfachte perspektivische Darstellung eines weiteren Ausführungsbeispiels, mit zwei gegenüberliegenden Klemmelementen in Form von expandierbaren elastischen Körpern, wobei dargestellt ist, wie ein durch die Vorrichtung zu haltender Gegenstand in diese eingebracht werden sollen,
- Fig. 4: die Darstellung des zweiten Ausführungsbeispiels bei der der Gegenstand durch die expandierten elastischen Körper gehalten ist,
- Fig. 5: eine Draufsicht auf die Darstellung von Fig. 4,
- Fig. 6: eine Darstellung eines dritten Ausführungsbeispiels einer erfindungsgemäßen Vorrichtung, bei denen die expandierbaren Körper eines Klemmelements in drei einzelne Körper unterteilt sind,
- Fig. 7: ein viertes Ausführungsbeispiel einer erfindungsgemäßen Vorrichtung mit einer noch weiteren Aufteilung in eine Vielzahl an expandierbaren Körpern,
- Fig. 8: ein fünftes Ausführungsbeispiel einer erfindungsgemäßen Vorrichtung, teilweise im Schnitt, wobei dargestellt ist, wie ein Schaft eines Gegenstandes zwischen zwei Klemmelementen in einer Grundstellung gehalten ist, wobei jedes Klemmelement zwei expandierbare elastische Körper aufweist,
- Fig. 9: eine der Fig. 8 vergleichbare Darstellung, wobei dargestellt ist, dass der Schaft aus Fig. 8 innerhalb der Vorrichtung seitlich versetzt ist, und
- Fig. 10: eine entsprechende Darstellung, bei der dargestellt ist, dass der in der Vorrichtung gehaltene Schaft auch verkippt werden kann.

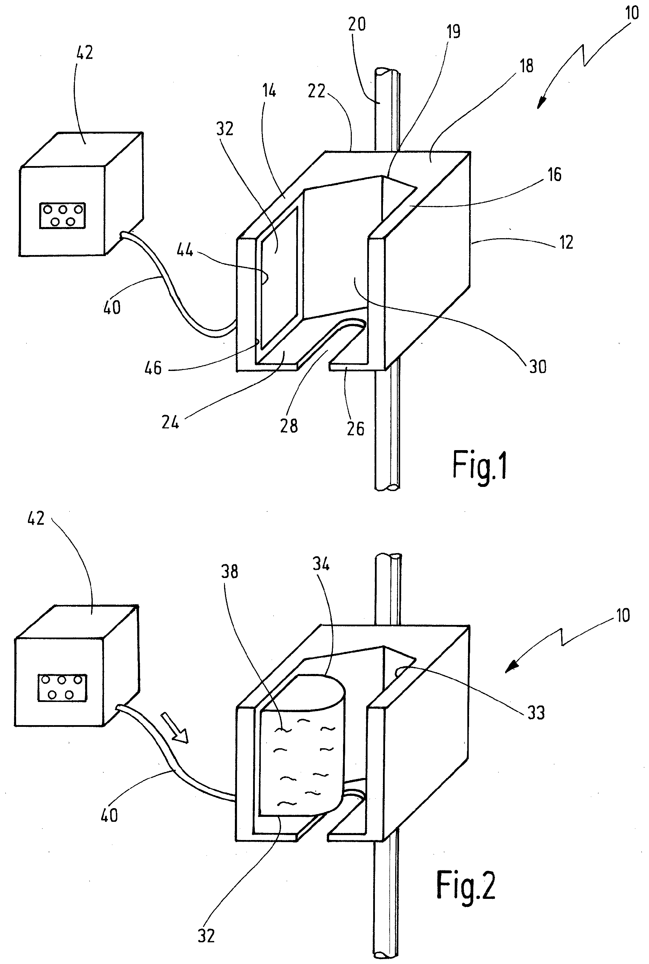

Ein in den Figuren 1 und 2 dargestelltes erstes Ausführungsbeispiel einer erfindungsgemäßen Vorrichtung, ist in seiner Gesamtheit mit der Bezugsziffer 10 bezeichnet.

Die Vorrichtung 10 weist einen massiven U-förmigen Grundkörper 12 auf. Dabei erstrecken sich zwei im Abstand voneinander angeordnete plattenförmige, sich parallel zueinander erstreckende Schenkel 14 und 16 des Grundkörpers 12 von einer, diese an einem Ende, verbindenden Wand 18 fort.

An der Innenseite ist die Wand 18 mit einer Kerbe 19 versehen.

An der äußeren oder Rückseite 22 der Wand 18, ist ein Ständer 20 angebracht, an dem der Grundkörper 12 befestigt ist. Je nachdem, ob die Vorrichtung transportabel ausgebildet ist, ist der Ständer 20 so ausgebildet, dass er auf einem Boden aufgestellt werden kann und der Grundkörper 12 an dem Ständer 20 in einer bestimmten Höhenstellung arretiert werden kann. Der Grundkörper 12 kann aber auch an einem teleskopartigen Arm oder dergleichen befestigt sein, der an irgendeiner Stelle einer Wand oder sonst irgendwo befestigt sein kann. Aus Fig. 1 und 2 ist zu erkennen, dass der Grundkörper 12 so angeordnet ist, dass die offene Seite des U sich dem Betrachter zuwendet.

An einem in Schwerkraftrichtung unteren Ende der Schenkel 14 und 16 ist eine Abrutschsicherung 24 angeordnet.

Die Abrutschsicherung 24 besteht aus einer unteren, die beiden Schenkel 14 und 16 verbinden Bodenplatte 26, die etwa mittig mit einem Schlitz 28 versehen ist.

Der Schlitz 28 in der Bodenplatte 26 öffnet zu der dem Betrachter zugewandten offenen Seite 30 des Grundkörpers 12. An der Innenseite 46 des Schenkels 14 ist ein Klemmelement 32 montiert. Das Klemmelement 32 ist als ein elastischer geschlossener Hohlkörper 34 aufgebaut, der in einer hier nicht näher bezeichneten Aussparung in dem Schenkel 14 an einer Seite haftend montiert ist. Der Hohlkörper 34 ist als eine Art Kissen 44 ausgestaltet.

Ein Fluid 38, sei es ein gasförmiges Fluid, bspw. Druckluft, oder eine Flüssigkeit, kann von einer Steuerung 42 in den Innenraum 36 des Kissens 44 eingeführt werden, wie das in Fig. 2 durch einen Pfeil dargestellt ist.

Dadurch expandiert das Kissen 44, wie das in Fig. 2 dargestellt ist, bewegt sich also in Richtung des gegenüberliegenden Schenkels 16, der hier als unbewegliches zweites Klemmelement 33 funktioniert.

Befindet sich ein hier nicht dargestellter Gegenstand zwischen der Innenseite des als stationäres Klemmelement 33 wirkenden Schenkels 16 und dem expandierenden Kissen 44, wird dieser Gegenstand zwischen die beiden Schenkel 14 und 16 eingeklemmt und ist in der Vorrichtung 10 gehalten.

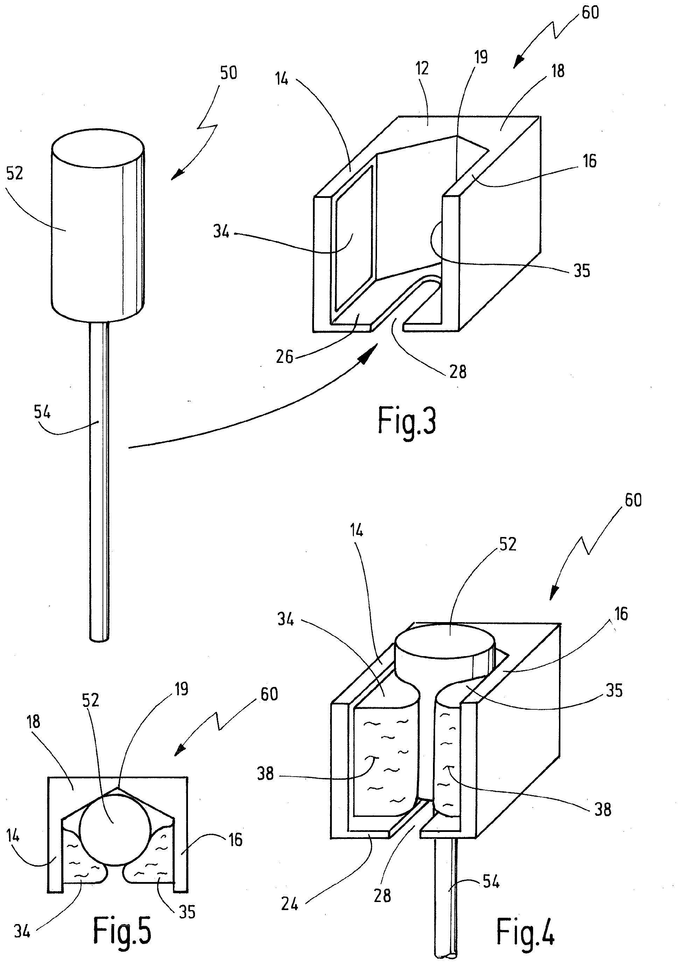

Dieser Vorgang soll anhand der Figuren 3 bis 5 in Zusammenhang mit einem zweiten Ausführungsbeispiel einer erfindungsgemäßen Vorrichtung 60 näher beschrieben und erläutert werden.

Die in Fig. 3 dargestellte Vorrichtung 60 ist im Grundprinzip gleich aufgebaut wie die Vorrichtung der Figuren 1 und 2, wobei der Übersichtlichkeit halber der Ständer 20 und die Steuerung 42 weggelassen wurde. Daher sind in den Figuren 3 bis 5 für an sich identische Bauteile auch dieselben Bezugszeichen eingesetzt worden.

Das heißt auch die Vorrichtung 60 weist einen U-förmigen Grundkörper 12 auf, der zwei parallele Schenkel 14 und 16 aufweist. An jeder Innenseite der Schenkel 14 und 16 ist hier ein Klemmelement in Form eines elastischen expandierbaren Hohlkörpers 34 bzw. 35 montiert, wie das in Zusammenhang in Fig. 1 bereits beschrieben worden ist. Selbstverständlich sind beide Hohlkörper 34 und 35 über Leitungen mit der Steuerung verbunden, so dass beide Hohlkörper 34 und 35 mit einem Fluid beaufschlagt und expandiert werden können.

In Fig. 3 ist dargestellt, wie ein Gegenstand 50 in die Vorrichtung 60 eingebracht werden soll.

Der Gegenstand 50 soll symbolisch einen Körper darstellen, der an einem Ende einen relativ großen bulkigen Kopf 52 aufweist, von dem sich ein langerstreckter durchmessergeringerer Schaft 54 forterstreckt.

Dieser Gegenstand steht bspw. symbolisch für ein medizinisches endoskopisches Instrument.

Wie aus Fig. 3 ersichtlich, kann der Gegenstand 50 bspw. seitlich in die Vorrichtung 60 so eingeführt werden, dass der Schaft 54 in den offenen Schlitz 28 eingeführt wird.

Es ist auch möglich, den Gegenstand 50 von oben in den Grundkörper 12 zwischen die Schenkel 14 und 16 einzuführen.

Sinnvollerweise wird der Gegenstand 50 so in die Vorrichtung 60 eingesetzt, dass er an der mit der Kerbe 19 versehen hinteren Wand 18 zum Liegen kommt, wie das insbesondere aus Fig. 5 ersichtlich ist.

Dabei kann sinnvollerweise, muss aber nicht zwingend so sein, das untere Ende des durchmessergrößeren Kopfes 52 auf der Bodenplatte 26 der Abrutschsicherung abgesetzt werden.

Bei Aktivierung der Hohlkörper 34 und 35, also bei Beaufschlagung mit Fluid 38, expandieren die elastischen Hohlkörper 34 und 35 und legen sich dabei an die Außenseite des Kopfes 52 des Gegenstandes 50 an und passen sich dessen Außenkontur an. Aus der Draufsicht von Fig. 5 ist ersichtlich, dass der Kopf 52 zum einen durch die beiden gegenüberliegenden aufgeblähten bzw. expandierten Hohlkörper 34 und 35 und durch die Wand 18 gehalten wird.

Hat der Gegenstand 50 diese Kreisgeometrie, ist ersichtlich, dass der Gegenstand 50 bspw. auch in dem fixierten Zustand um seine Längsachse verdreht werden kann. Es ist auch möglich, den Gegenstand 50 etwas in Richtung dessen Schaftachse anzuheben, falls das erwünscht ist.

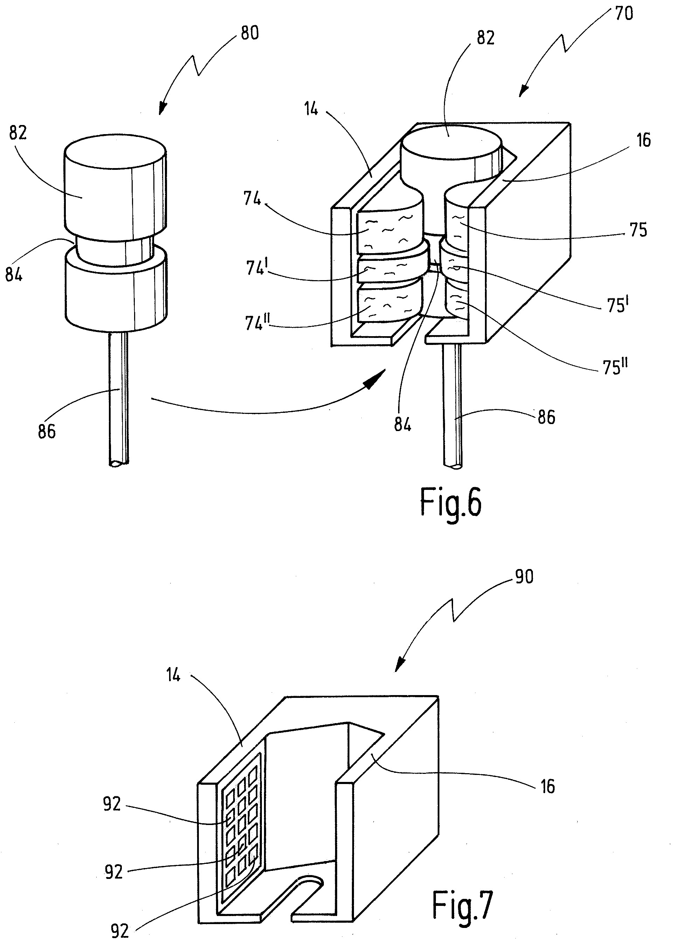

Bei dem in Fig. 6 dargestellten dritten Ausführungsbeispiel einer erfindungsgemäßen Vorrichtung 70 ist diese wiederum, was den Grundkörper und die Schenkel 14 und 16 betrifft, gleich ausgebildet, wie die zuvor beschriebenen Ausführungsbeispiele. Auch bei der Vorrichtung 70 sind an den Innenseiten beider Schenkel 14 und 16 expandierbare Hohlkörper als Klemmelemente angeordnet.

An der Innenseite des Schenkels 14 sind drei übereinander angeordnete und voneinander getrennte Hohlkörper 74, 74' und 74" angeordnet. Diesen exakt gegenüberliegend, sind an der Innenseite des Schenkels 16 entsprechend drei voneinander getrennte Hohlkörper 75, 75' und 75" angeordnet. Jeder der einzelnen Hohlkörper 74, 74', 74" und 75, 75' und 75", ist über eine separate Versorgungsleitung mit der hier nicht dargestellten Steuerung verbunden, d.h. jeder einzelne der Hohlkörper kann individuell mit einem Fluid beaufschlagt werden.

Als Erläuterung ist in Fig. 6 ein Gegenstand 80 dargestellt, dessen großer bulkiger Kopf eine Einschnürung 84 aufweist. Auch hier erstreckt sich ein Schaft 86 vom Kopf 82 weg.

Ist dieser Gegenstand 80 in die Vorrichtung 70 eingeschoben und werden die Hohlkörper aufgebläht, so kann sich der jeweils mittlere der Hohlkörper, nämlich 74' und 75' etwas weiter in den Gegenstand hinein erstrecken, bspw. genau in die Einschnürung 84. Dies demonstriert die flexible und fein abstimmbare Anpassung der flexiblen Hohlkörper, um ein effektives und wirksames Halten eines Gegenstandes 80 zu erzielen.

In Fig. 7 ist ein viertes Ausführungsbeispiel einer erfindungsgemäßen Vorrichtung 90 dargestellt, die ebenfalls wieder prinzipiell wie die zuvor dargestellten Vorrichtungen ausgestaltet ist, wobei hier auf einer oder beiden Seiten der Schenkel 14 und 16, ein Muster einer Vielzahl an elastischen expandierbaren Hohlkörpern 92 angeordnet ist.

Bei dem in Fig. 7 dargestellten Ausführungsbeispiels, liegen auf einer Höhe drei solche Körper nebeneinander und außerdem jeweils fünf solche Körper übereinander.

Dies ist selbstverständlich nur ein ausgewähltes Ausführungsbeispiel und kann entsprechend variiert werden. Je nach Größe und Ausgestaltung können alle Hohlkörper einzeln angesteuert und mit Fluid beaufschlagt werden, oder es können auch einzelne oder mehrere Gruppen angesteuert werden, je nachdem, wie das erwünscht ist. Dadurch ist eine besonders gute Anpassung an unterschiedlich geformte Außenseiten von zu haltenden Gegenständen möglich.

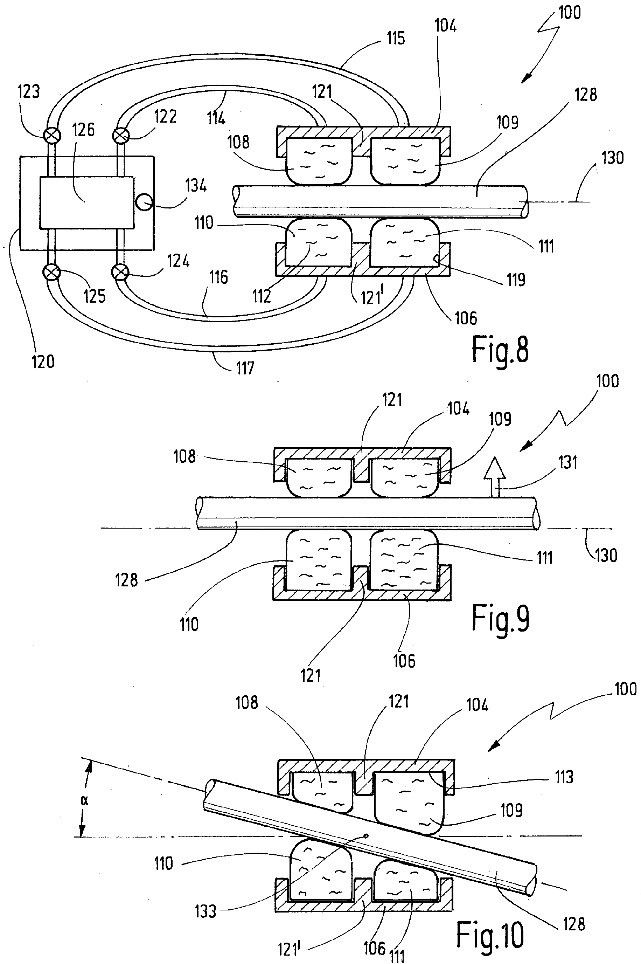

In den Figuren 8 bis 10 ist eine weitere Ausgestaltung im Rahmen eines fünften Ausführungsbeispiels einer erfindungsgemäßen Vorrichtung 100 dargestellt.

Aus der Schnittdarstellung von Fig. 8 ist ersichtlich, dass hier wieder zwei Schenkel 104 und 106 vorhanden sind, die sich parallel und im Abstand zueinander erstrecken.

An der Innenseite des Schenkels 104 sind zwei elastisch verformbare Klemmelemente montiert, die als Kissen 108 und 109 ausgebildet sind. Auf dem gegenüberliegenden Schenkel 106 sind entsprechend zwei Kissen 110, 111 montiert. Dazu weist der Schenkel 104 an seiner Innenseite einen entsprechenden Sitz 113 auf, am Schenkel 106 ist ein entsprechender Sitz 119 vorhanden. Jeder Sitz weist einen Rahmen 121, 121' auf, in dem das entsprechende Kissen gehalten und geführt ist. Jedes der Kissen 108, 109, 110, 111 ist über eine separate Leitung 114, 115, 116, 117 mit einer Steuerung 120 verbunden. Im Pfad jeder Leitung 114 - 117 ist noch ein Ventil 122 - 125 angeordnet, die alle einzeln und unabhängig voneinander angesteuert werden können.

Alle Leitungen 114 - 117 werden aus einem gemeinsamen Vorratsgefäß 126 mit einem Fluid 112, hier eine Flüssigkeit, gespeist.

In Fig. 8 ist ein Zustand dargestellt, bei dem alle vier Kissen 108 - 111 gleichermaßen mit dem Fluid 112 beaufschlagt sind. Dadurch ist der Schaft 128 mittig in einer Ausgangslage 130 gehalten. In der Darstellung von Fig. 8 ist das eine horizontale Lage, es kann selbstverständlich auch eine entsprechend vertikal oder schräg ausgerichtete Lage sein.

In Fig. 9 ist dargestellt, dass die beiden Kissen 108 und 109 etwas kollabiert sind, wohingegen die Kissen 110, 111 weiter expandiert bzw. aufgebläht sind.

Dies ist dadurch erfolgt, dass entsprechend der Darstellung von Fig. 8 aus den Kissen 108, 109 über die Leitungen 114, 115 durch die Steuerung 120 eine gewisse, und zwar jeweils dieselbe Menge an flüssigem Fluid 112 abgeführt wurde, und zwar in das gemeinsame Vorratsgefäß 126. Gleichzeitig wurde in die Kissen 110, 111 eine entsprechend größere Menge an Fluid 112 über die Steuerung 120 und die Leitung 116, 117 eingepresst.

Dadurch hat sich der Schaft 128, wie das aus dem Übergang von Fig. 8 zu Fig. 9 ersichtlich ist, aus seiner Ausgangslage 130 bewegt, in der Darstellung hat er sich nach oben bewegt, wie das durch einen Pfeil 131 dargestellt ist.

Daraus wird ersichtlich, dass ein in der Vorrichtung 100 gehaltener Gegenstand in einem gewissen Ausmaß bewegt werden kann, wobei er aber als solcher fest gehalten ist.

In Fig. 10 ist dargestellt, wie sich der Schaft 128 aus seiner Ausgangslage 130 durch eine Kippbewegung bewegt hat.

Dazu wurde etwas Fluid aus dem Kissen 108 abgeführt und dem Kissen 109 etwas mehr Fluid zugeführt.

Dementsprechend wurde in dem dem Kissen 108 gegenüberliegenden Kissen 110 entsprechend Fluid zugeführt und, aus dem Kissen 111 entsprechend, Fluid abgeführt. Dies führt zu einem Drehen oder Verkippen des Schaftes 128 um einen Drehpunkt 133.

Es ist einleuchtend, dass auch beide Verschiebebewegungen kombiniert werden können, also dass der Schaft 128 sowohl aus seiner Ausgangslage 130 angehoben oder abgesenkt, oder bei vertikaler Ausrichtung, links oder rechts versetzt wurde und zugleich eine Schwenkbewegung durchgeführt wird.

Dies ist dadurch möglich, dass jedes einzelne Kissen 108 - 111 individuell über die entsprechenden Leitungen 114 - 117 angesteuert werden kann, also mit zusätzlichem Fluid beaufschlagt werden kann und somit expandiert, oder, dass aus dem entsprechenden Kissen Fluid abgezogen wird, und dieses dann entsprechend kollabiert.

Dies ermöglicht der Handhabungsperson bei fest in der Vorrichtung 100 gehaltenem Gegenstand dennoch gewisse Ausrichtungen oder Lageveränderungen durchzuführen. Somit führt hier die Vorrichtung nicht nur eine reine Haltefunktion durch, sondern stellt auch eine, in gewissem Maße, Ausricht- oder Positioniervorrichtung dar.

In Fig. 8 ist dargestellt, dass die Steuerung 120 ein Steuerelement 134 aufweist. Durch Betätigung des Steuerelementes 134 können alle vier Kissen 108 - 111 zugleich entspannt werden und somit kollabieren, so dass durch eine einzige Betätigung die Klemmwirkung aufgehoben werden kann und der Gegenstand, sprich der Schaft 128, aus der Vorrichtung 100 entnommen werden kann. Dadurch, dass alle Kissen aus einem einzigen Vorratsbehälter versorgt werden, ist ein in sich abgeschlossenes Flüssigkeitssystem vorhanden, so dass keine Gefahr besteht, dass Kontaminationen, wenn die Vorrichtung im medizinischen Bereich Einsatz findet, in das Fluid dringen können.

Wie eingangs erläutert, ist es möglich, sowohl die Vorrichtung als auch den Gegenstand, also den Schaft 128, mit einem Drape zu überziehen, die Funktionsweise der Haltevorrichtung wird dadurch nicht beeinträchtigt.

Ist das Fluid ein gasförmiges Fluid, wird üblicherweise Druckluft herangezogen, die entweder aus einem Druckluftbehälter stammt, oder Vorort in der Steuerung durch einen Kompressor zur Verfügung gestellt wird.

## Patentansprüche

1. Vorrichtung zum zeitweiligen Halten eines Gegenstandes (50, 80, 128), der ein relativ durchmessergroßes Hand- oder Kopfstück (52) aufweist, von dem sich ein langerstreckter durchmessergeringerer Schaft (54) forterstreckt, insbesondere zum Halten eines medizinischen Instrumentes, mit zumindest zwei Klemmelementen (32, 33) zwischen die der Gegenstand (50, 80, 128) bringbar ist, wobei zumindest ein Klemmelement (32) auf das andere Klemmelement (33) zu bewegbar ist, so dass der Gegenstand (50, 80, 128) zwischen den Klemmelementen (32, 33) führbar und/oder fixierbar ist, wobei das zumindest eine bewegbare Klemmelement (32) als expandierbarer elastischer Körper (34, 35; 74, 74', 74"; 75, 75', 75"; 92) ausgebildet ist, der mit einem Fluid (38, 112) beaufschlagbar ist, und mit einer Steuerung (42, 120) zum Steuern der Beaufschlagung des zumindest einen Körpers (34, 35; 74, 74', 74"; 75, 75', 75"; 92) mit dem Fluid (38, 112) zum Expandieren bzw. Kollabieren des Körpers (34, 35; 74, 74', 74"; 75, 75', 75"; 92), wobei sich der expandierte Körper (34, 35; 74, 74', 74"; 75, 75', 75"; 92) aufgrund seiner Elastizität um Bereiche der Außenseite des zu haltenden Gegenstandes (50, 80, 128) anschmiegt und sich somit dessen Außenkontur anpasst, **dadurch gekennzeichnet, dass**, bei nicht expandiertem Klemmelement, ein Abstand zwischen den Klemmelementen (32, 33) derart ist, dass auch das durchmessergrößere Hand- oder Kopfstück (52) des zu haltenden Gegenstandes (50, 80, 128) zwischen die Klemmelemente bringbar ist, und dass, in Schwerkraftrichtung gesehen, unter den Klemmelementen (32, 33) eine Abrutschsicherung vorhanden ist, die, bei nicht expandiertem Klemmelement, ein Abrutschen des Gegenstandes (50, 80, 128) von der Vorrichtung dadurch sperrt, dass das Hand- oder Kopfstück (52) zurückhaltbar ist.

2. Vorrichtung nach Anspruch 1, **dadurch gekennzeichnet, dass** der Körper (34, 35; 74, 74', 74"; 75, 75', 75"; 92) als Hohlkörper (34, 35) ausgebildet ist, dessen Innenraum (36) mit dem Fluid (38, 112) beaufschlagbar ist.

3. Vorrichtung nach Anspruch 1 oder 2, **dadurch gekennzeichnet, dass** der expandierbare Körper (34, 35; 74, 74', 74"; 75, 75', 75"; 92) als Kissen (108, 109, 110, 111) ausgebildet ist, das an einem Sitz (113, 119) in einem Rahmen (121, 121') gehalten und/oder geführt ist.

4. Vorrichtung nach einem der Ansprüche 1 bis 3, **dadurch gekennzeichnet, dass** beide Klemmelemente (32, 33) als expandierbare elastische Körper (34, 35; 74, 74', 74"; 75, 75', 75"; 92) ausgebildet sind.

5. Vorrichtung nach Anspruch 4, **dadurch gekennzeichnet, dass** die beiden expandierbaren elastischen Körper (34, 35; 74, 74', 74"; 75, 75', 75"; 92) an gegenüberliegenden, im Abstand voneinander angeordneten Sitzen (113, 119) befestigt sind.

6. Vorrichtung nach einem der Ansprüche 1 bis 5, **dadurch gekennzeichnet, dass** jeder expandierbare elastische Körper aus mehreren einzelnen, expandierbaren elastischen Körpern (74, 74', 74"; 75, 75', 75"; 92) aufgebaut ist.

7. Vorrichtung nach Anspruch 6, **dadurch gekennzeichnet, dass** jeder der mehreren einzelnen expandierbaren elastischen Körper, unabhängig von einem anderen, über die Steuerung (42, 120) mit dem Fluid (38, 112) beaufschlagbar ist.

8. Vorrichtung nach einem der Ansprüche 1 bis 7, **dadurch gekennzeichnet, dass** diese einen U-förmigen Grundkörper (12) aufweist und dass die beiden Klemmelemente (32, 33) an gegenüberliegenden Innenseiten der Schenkel (14, 16; 104, 106) des U angebracht sind.

9. Vorrichtung nach Anspruch 8, **dadurch gekennzeichnet, dass** der U-förmige Grundkörper (12) eine Abrutschsicherung (24) in Form einer in Schwerkraftrichtung gesehen, unteren Platte (26) aufweist, die eine Öffnung enthält, durch die schaftartige Bauteile (54, 86) eines Gegenstandes (50, 80) hindurchreichen, durchmessergrößere Bauteile (52, 82) jedoch zurückhaltbar sind.

10. Vorrichtung nach Anspruch 9, **dadurch gekennzeichnet, dass** die Öffnung als zur offenen Seite des U's des Grundkörpers (12) öffnender Schlitz (28) ausgebildet ist.

11. Vorrichtung nach einem der Ansprüche 8 bis 10, **dadurch gekennzeichnet, dass** die Schenkel (14, 16) des Grundkörpers (12) durch eine Wand (18) geschlossen sind.

12. Vorrichtung nach einem der Ansprüche 1 bis 11, **dadurch gekennzeichnet, dass** alle mit dem Fluid (38, 112) beaufschlagbare expandierbare elastische Körper (34, 35; 74, 74', 74"; 75, 75', 75"; 92) über Leitungen (114-117) mit einer einzigen Quelle (126) des Fluids (38, 112) verbunden sind.

13. Vorrichtung nach Anspruch 12, **dadurch gekennzeichnet, dass** durch die Steuerung (120) über Ventile (122-125) jeder der elastischen Körper, unabhängig von einem anderen, mit dem Fluid (38, 112) beaufschlagbar ist.

14. Vorrichtung nach einem der Ansprüche 1 bis 13, **dadurch gekennzeichnet, dass** die Steuerung (120) ein Steuerelement (134) aufweist, über das alle expandierbaren Körper zugleich kollabierbar sind.

## Claims

1. Device for temporarily holding an object (50, 80, 128) comprising a hand or head piece (52) having a relatively large diameter, wherein an elongated shaft (54), having a smaller diameter, extends from the hand or head piece (52), particularly for holding a medical instrument, the device comprising two clamping elements (32, 34) between which the object (50, 80, 128) can be brought, wherein at least one clamping element (32) is arranged to be moved towards the other clamping element (33) so that the object (50, 80, 128) can be led and/or fixed between the clamping elements (32, 33), wherein the at least one movable clamping element (32) is arranged as an expandable elastic body (34, 35; 74, 74', 74", 75, 75', 75"; 92) to which a fluid (38, 112) may be applied, and comprising a controller (42, 120) for controlling the application of the fluid (38, 112) to the at least one body (34, 35; 74, 74', 74", 75, 75', 75"; 92) for expanding and/or collapsing the body (34, 35; 74, 74', 74", 75, 75', 75"; 92), wherein the expanded body (34, 35; 74, 74', 74", 75, 75', 75"; 92), due to its elasticity, nestles about exterior regions of the object (50, 80, 128) to be held and thus adapts itself to the outer contour thereof, **characterized in that** with the clamping element not expanded, a distance between the clamping elements (32, 33) is such that also the hand or head piece (52), having a larger diameter, of the object (50, 80, 128) to be held may be brought between the clamping elements, and that, as seen in the direction of gravity, a slipping off prevention feature is provided beneath the clamping elements (32, 33) that, with the clamping element not expanded, blocks a slipping off of the object (50, 80, 128) from the device, since the hand or head piece (52) is retainable.

2. Device according to Claim 1, **characterized in that** the body (34, 35; 74, 74', 74"; 75, 75', 75"; 92) is arranged as a hollow body (34, 35) having an interior (36) to which the fluid (38, 112) can be applied.

3. Device according to claim 1 or 2, **characterized in that** the expandable body (34, 35; 74, 74', 74"; 75, 75', 75"; 92) is arranged as a cushion (108, 109, 110, 111), which is held and/or guided on a seat (113, 119) in a frame (121, 121').

4. Device according to anyone of claims 1 to 3, **characterized in that** both clamping elements (32, 33) are arranged as expandable elastic bodies (34, 35; 74, 74', 74"; 75, 75', 75"; 92).

5. Device according to claim 4, **characterized in that** the two expandable elastic bodies (34, 35; 74, 74', 74"; 75, 75', 75"; 92) are attached to opposite seats (113, 119) arranged at a distance from one another.

6. Device according to anyone of claims 1 to 5, **characterized in that** each expandable elastic body is composed of several individual expandable elastic bodies (74, 74', 74"; 75, 75', 75"; 92).

7. Device according to claim 6, **characterized in that** the fluid (38, 112) can be applied to each of the several individual expandable elastic bodies, independently of one another, via the control system (42, 120).

8. Device according to anyone of claims 1 to 7, **characterized in that** the device comprises a U-shaped main body (12), and **in that** the two clamping elements (32, 33) are mounted on opposite inner faces of the arms (14, 16; 104, 106) of the U.

9. Device according to claim 8, **characterized in that** the U-shaped main body (12) comprises a slipping off prevention feature (24) arranged as a lower plate (26), as seen in the direction of gravity, wherein the lower plate (26) comprises an opening through which shaft-like components (54, 86) of an object (50, 80) extend, while components (52, 82) of greater diameter can be retained.

10. Device according to claim 9, **characterized in that** the opening is arranged as a slot (28) opening itself towards the open side of the U of the main body (12).

11. Device according to anyone of claims 8 to 10, **characterized in that** the arms (14, 16) of the main body (12) are completed by a wall (18).

12. Device according to anyone of claims 1 to 11, **characterized in that** each of the expandable elastic bodies (34, 35; 74, 74', 74"; 75, 75', 75"; 92) to which the fluid (38, 112) can be applied are connected via lines (114 - 177) to a single source (126) of the fluid (38, 112).

13. Device according to claim 12, **characterized in that**, by means of the control system (120), the fluid (38, 112) can be applied to each of the elastic bodies, independently of one another, via valves (122 - 125).

14. Device according to anyone of claims 1 to 13, **characterized in that** the control system (120) comprises a control element (134) via which each of the expandable bodies are collapsible simultaneously.

## Revendications

1. Dispositif de maintien intermittent d'un objet (50, 80, 128), qui présente une pièce à main ou une pièce de tête (52) de relativement grand diamètre, prolongée par une tige (54) de plus faible diamètre et plus allongée, en particulier pour maintenir un instrument médical, comprenant au moins deux éléments de serrage (32, 33) entre lesquels l'objet (50, 80, 128) peut être amené, au moins un élément de serrage (32) pouvant être déplacé vers l'autre élément de serrage (33), de telle sorte que l'objet (50, 80, 128) puisse être guidé et/ou fixé entre les éléments de serrage (32, 33), l'au moins un élément de serrage déplaçable (32) étant réalisé sous forme de corps élastique expansible (34, 35 ; 74, 74', 74" ; 75, 75', 75" ; 92), qui peut être sollicité avec un fluide (38, 112), et comprenant une commande (42, 120) pour commander la sollicitation de l'au moins un corps (34, 35 ; 74 74', 74" ; 75, 75', 75"; 92) avec le fluide (38, 112) pour dilater et/ou contracter le corps (34, 35 ; 74, 74', 74" ; 75, 75', 75" ; 92), le corps dilaté (34, 35 ; 74, 74', 74" ; 75, 75', 75" ; 92), en raison de son élasticité, s'appliquant étroitement contre et autour des régions du côté extérieur de l'objet à maintenir (50, 80, 128) et par conséquent à son contour extérieur, **caractérisé en ce que** lorsque l'élément de serrage n'est pas dilaté, une distance entre les éléments de serrage (32, 33) est telle que la pièce à main ou la pièce de tête (52) de plus grand diamètre de l'objet à maintenir (50, 80, 128) puisse être amenée entre les éléments de serrage, et que, vu dans la direction de la pesanteur, une fixation anti-glissement est prévue sous les éléments de serrage (32, 33), laquelle bloque un glissement de l'objet (50, 80, 128) du dispositif lorsque l'élément de serrage n'est pas dilaté, de telle sorte que la pièce à main ou la pièce de tête (52) puisse être retenue.

2. Dispositif selon la revendication 1, **caractérisé en ce que** le corps (34, 35 ; 74, 74', 74" ; 75, 75', 75" ; 92), est réalisé sous forme de corps creux (34, 35) dont l'espace interne (36) peut être sollicité avec le fluide (38, 112).

3. Dispositif selon la revendication 1 ou 2, **caractérisé en ce que** le corps expansible (34, 35 ; 74, 74', 74" ; 75, 75', 75" ; 92) est réalisé sous forme de coussin (108, 109, 110, 111) qui est retenu et/ou guidé au niveau d'un siège (113, 119) dans un cadre (121, 121').

4. Dispositif selon l'une quelconque des revendications 1 à 3, **caractérisé en ce que** les deux éléments de serrage (32, 33) sont réalisés sous forme de corps élastique expansible (34, 35 ; 74, 74', 74" ; 75, 75', 75" ; 92).

5. Dispositif selon la revendication 4, **caractérisé en ce que** les deux corps élastiques expansibles (34, 35 ; 74, 74', 74" ; 75, 75', 75" ; 92) sont fixés au niveau de sièges opposés (113, 119) disposés à distance l'un de l'autre.

6. Dispositif selon l'une quelconque des revendications 1 à 5, **caractérisé en ce que** chaque corps élastique expansible est constitué de plusieurs corps élastiques expansibles individuels (74, 74', 74" ; 75, 75' ; 75" ; 92).

7. Dispositif selon la revendication 6, **caractérisé en ce que** chacun des plusieurs corps élastiques expansibles individuels, indépendamment les uns des autres, peut être sollicité par le biais de la commande (42, 120) avec le fluide (38, 112).

8. Dispositif selon l'une quelconque des revendications 1 à 7, **caractérisé en ce que** celui-ci présente un corps de base en forme de U (12) et **en ce que** les deux éléments de serrage (32, 33) sont montés au niveau de côtés intérieurs opposés des branches (14, 16 ; 104, 106) du U.

9. Dispositif selon la revendication 8, **caractérisé en ce que** le corps de base en forme de U (12) présente une fixation anti-glissement (24) en forme de plaque inférieure (26), vue dans la direction de la pesanteur, laquelle contient une ouverture à travers laquelle pénètrent des composants de type tiges (54, 86) d'un objet (50, 80) tandis que des composants de plus grand diamètre (52, 82) sont retenus.

10. Dispositif selon la revendication 9, **caractérisé en ce que** l'ouverture est réalisée sous forme de fente (28) s'ouvrant vers le côté ouvert du U du corps de base (12).

11. Dispositif selon l'une quelconque des revendications 8 à 10, **caractérisé en ce que** les branches (14, 16) du corps de base (12) sont fermées par une paroi (18).

12. Dispositif selon l'une quelconque des revendications 1 à 11, **caractérisé en ce que** tous les corps élastiques expansibles (34, 35 ; 74, 74', 74" ; 75, 75', 75" ; 92) pouvant être sollicités avec le fluide (38, 112) sont connectés par le biais de conduites (114-117) à une source unique (126) de fluide (38, 112).

13. Dispositif selon la revendication 12, **caractérisé en ce que** chacun des corps élastiques, indépendamment des autres, peut être sollicité avec le fluide (38, 112) par la commande (120) par le biais de soupapes (122-125).

14. Dispositif selon l'une quelconque des revendications 1 à 13, **caractérisé en ce que** la commande (120) présente un élément de commande (134) par le biais duquel tous les corps expansibles peuvent être rétractés en même temps.
